# EUROPEAN PATENT APPLICATION

(11) **EP 3 146 962 A1**
(43) Date of publication of application: **29.03.2017**
(21) Application number: 15795688.9
(22) Date of filing: 20.05.2015
(51) Int. Cl.: A61K 9/14, A61K 31/4178, A61K 47/38, A61K 47/32, A61K 47/34, A61P 9/12

(54) **ALLISARTAN ISOPROXIL SOLID DISPERSION AND PHARMACEUTICAL COMPOSITION THEREOF**

(30) Priority: 23.05.2014 CN 201410223097
(71) Applicant: Shenzhen Salubris Pharmaceuticals Co., Ltd, Shenzhen, Guangdong 518040 (CN)
(72) Inventor: YE, Guanhao, Shenzhen Guangdong 518040 (CN); BU, Shui, Shenzhen Guangdong 518040 (CN)
(74) Representative: Schollweck, Susanne
(86) International application number: PCT/CN2015/079352
(87) International publication number: WO 2015/176655

(57) **Abstract**

The present invention provides an allisartan isoproxil solid dispersion with high drug loading and stability. On that basis, it also provides an allisartan isoproxil pharmaceutical composition containing mentioned solid dispersion. Compared with available technologies, the mentioned allisartan isoproxil pharmaceutical composition is characterized by high stability, good dissolution performance, and improved patient compliance, etc.

## Description

### Field of the Invention

The present invention belongs to the field of pharmaceutical chemistry, in particular, it relates to allisartan isoproxil solid dispersion and pharmaceutical composition containing the solid dispersion.

### Background of the Invention

Allisartan isoproxil (CAS: 947331-05-7), with the chemical name: 2-butyl-4-chloro - 1- [2'- (1H- tetrazol-5-yl) -1, 1'-biphenyl - methyl] - imidazole-5-carboxylic acid, 1 - [(isopropoxy) - carbonyl oxy] -, methyl ester, is a novel angiotensin II receptor antagonist. Chinese patent CN200680000397.8 discloses the structure of allisartan isoproxil, which is with low toxicity, and better antihypertensive effect than products of the same type (such as losartan). It plays its antihypertensive effect by generating to active metabolite (EXP3174) by metabolism in vivo. Allisartan isoproxil is ester derivative of EXP3174 and its solubility in water is low, therefore, the resulting formulation using conventional formulation methods cannot meet the needs of clinical medication.

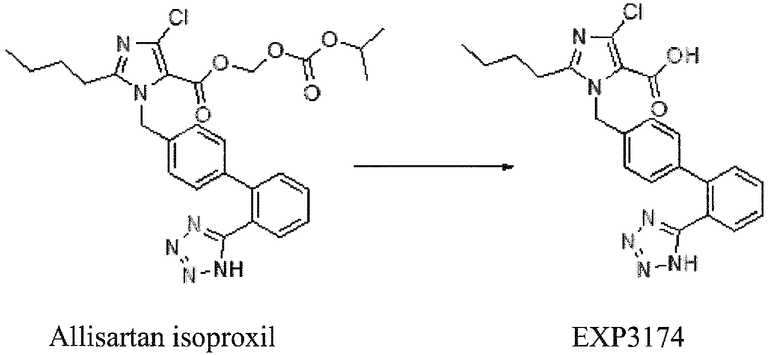

In order to overcome the low solubility of allisartan isoproxil, Chinese patent CN200880001668.0 provides a pharmaceutical composition of allisartan isoproxil by solid dispersion technology, in which carrier materials are selected from the group of polyethylene glycol, povidone, surfactant containing polyoxyethylene base, watersoluble cellulose derivatives, organic acids and sugar, sterols solubilizer. Addition of the solubilizer and use of solid dispersion technology can increase the dissolution of active ingredient effectively. Example D1-D6 shows that the dissolutions in 45 minutes are all more than 90% which meet the requirements of clinical medication. However, there are shortcomings in the preparation process of available technology, e.g., drug loading in the mentioned solid dispersion is not high, so content of active ingredient in the pharmaceutical composition is low, and the unit weight of preparation is too large, thus leading to poor patient compliance, which fails to achieve the optimization of clinical administration and antihypertensive effect.

Therefore, on the premise of ensuring stability and dissolution of preparation, increasing the active ingredient content and reducing the unit weight of preparation remain unresolved problems in the prior art. The present invention, started from the shortcomings of available technologies, discovered a new allisartan isoproxil solid dispersion with high drug loading, good dissolution and high stability, but reduced unit weight of the preparation after a large number of experiments.

### Summary of the Invention

The object of the present invention is to overcome the shortcomings of available technologies. On the premise of ensuring the stability and dissolution of the preparation, active ingredient content in the solid dispersion is effectively increased through adjusting the type and ratio of carrier materials, further a solid dispersion of allisartan isoproxil with high drug loading is discovered. The drug loading in the solid dispersion is higher than in those with the available technologies, and pharmaceutical composition containing the mentioned solid dispersion shows good dissolution, high stability, etc. which can meet the requirements of clinical medication. Finally, the unit weight of the preparation is reduced, and patient compliance is improved.

The above-described advantages of the mentioned solid dispersions are achieved by the following technical means:
An allisartan isoproxil solid dispersion is composed of allisartan isoproxil and pharmaceutically acceptable carrier materials. The mentioned carrier materials comprise solubilizing carrier, wherein the mass ratio of allisartan isoproxil to the solubilizing carrier in mentioned allisartan isoproxil solid dispersion is 1: 0.2 to 0.45. It is known in the field, solid dispersions refer to disperse active ingredient in carrier materials in the form of microcrystalline, amorphous or molecule through preparation methods, thereby improving the dissolution. Drug loading, i.e the amount of drug loading, refers to the loading amount of active ingredient in unit weight of carrier in solid dispersion. In general, the increasing amount of carrier materials used in the solid dispersions can improve drug dissolution, however, when the amount of carriers is increased up to certain extent, it will no longer obviously improve the drug dissolution. In addition, too much carrier materials can raise the cost, also increase the unit weight of the preparation. Usually, in order to achieve good solubilizing effect, the mass ratio of active ingredient to the carrier materials in solid dispersion is 1:5 ∼ 20, but for specific drugs, there is still some space to increase the drug loading of solid dispersion. Therefore, in order to improve the dissolution performance, optimum combination of active ingredient and carrier materials and their optimum ratio should be studied during the preparation of solid dispersion, meanwhile weight of preparation is controlled, further clinical medication and antihypertensive effect optimization are achieved, and finally the compliance is improved. However, it is difficult to prepare a solid dispersion with high dissolution and much drug loading. For the ration selection, too much drug loading leads to too little carriers used, so that active ingredient cannot keep in stable high dispersion state, thus it's unable to obtain solid preparation, which appears as unqualified dissolution. In addition, the type of carrier material can also affect the dispersion of active ingredients. The mentioned solubilizing carrier is selected from one or a mixture of two or more in any ratio of povidone, copovidone and other vinylpyrrolidone homopolymers or copolymers; polyvinyl alcohol; polyethylene glycol (PEG4000, PEG6000); methylcellulose, hydroxypropyl cellulose, hydroxypropyl methyl cellulose and other cellulose ethers; Eugragit L 100 and S100, acrylic resin II, acrylic resin III and other acrylic polymers; one or mixture of two or more in any ratio of hydroxypropyl methyl cellulose phthalate (HPMCP HP-55), cellulose acetate phthalate (CAP), hydroxypropyl methyl cellulose acetate succinate (HPMCAS), etc., preferably one or mixture of two or more in any ratio of povidone, copovidone, polyethylene glycol (PEG4000, PEG6000), hydroxypropyl cellulose, hydroxypropyl methyl cellulose, hydroxypropyl methyl cellulose phthalate (HPMCP HP-55), etc.; The mass ratio of allisartan isoproxil to solubilizing carrier is 1: 0.2 to 0.45, preferably 1: 0.30 to 0.40.

Wherein mentioned povidone is 1-vinyl-2-pyrrolidinone homopolymer. It can be classified by average molecular weight as PVP k12, PVP k15, PVP k17, PVP k25, PVP k30, PVP k29/32, PVP k60, PVP k120, etc., preferably PVP k29/ 32. The mentioned hydroxypropyl cellulose can be classified by average molecular weight as HPC-SSL, HPC-SL, HPC-L, HPC-M, HPC-H, etc., preferably HPC-SL. The mentioned hydroxypropyl methyl cellulose can be classified by viscosity as E3, E5, E6, E15, E50Lv, etc., preferably HPMC E6.

Preferably, the carrier materials in the allisartan isoproxil solid dispersion further include excipients, which can be selected from one or more than one mixed in any ratio of disintegrating agent, filler, binder and other pharmaceutical excipients except solubilizing carriers. The role of excipients is to carry the active ingredient and solubilizing carriers, so that active ingredient can be dispersed more stably and uniformly in solid dispersion in the form of microcrystal, amorphous or molecule, meanwhile the disintegration and dissolution of the pharmaceutical composition containing solid dispersion can be improved. Specifically, the excipients are selected from one or mixture in any ratio of more than one of cross-linked povidone, cross-linked sodium carboxymethyl cellulose, low-substituted hydroxypropyl cellulose, sodium carboxymethyl starch, microcrystalline cellulose, starch, pre-gelatinized starch, lactose, dextrin, mannitol, calcium sulfate, calcium phosphate, calcium hydrogen phosphate, etc., preferably cross-linked povidone. Mentioned cross-linked povidone is synthetic crosslinked N- vinyl-2-pyrrolidone homopolymer, such as PVPP XL, PVPP XL-10, etc.

Similarly, in order to achieve the purpose of carrying, dispersion, stabilizing of excipients, the amount of excipients needs to be screened. Too little excipients cannot play the role of carrying and stabilizing active ingredient in solid dispersion, while too much will make the preparation too large; also make the preparation process complicated. Specifically, the mass ratio of the allisartan isoproxil to the excipients is 1:0.10∼1.00, preferably 1:0.30∼0.80.

Preferably, the mass ratio of the allisartan isoproxil to the solubilizing carrier and excipients is 1:0.3∼0.4:0.3∼0.8.

The allisartan isoproxil solid dispersion mentioned can be prepared using conventional manufacturing methods in the field, such as solvent method, solvent deposition method, spray drying method, fluidized bed method, freeze drying method, melting extrusion method, preferably fluidized bed method.

For a preferred embodiment of the present invention, allisartan isoproxil solid dispersion is prepared by fluidized bed method with the following steps:
1. Dissolve solubilizing carrier in an appropriate amount of organic solvent, and then dissolve allisartan isoproxil in the above solution, mix well to give drug solution;
2. Place the excipients in the fluidized bed, and spray drug solution from top to form granulation, and finally get allisartan isoproxil solid dispersion.

A preferred technical solution of the solid dispersion in the present invention, and the compositions are as follows:

| Components | Content (unit) |
|---|---|
| Allisartan isoproxil | 1 |
| Povidone K29/32 | 0.35 |
| Crosslinked povidone | 0.35 |

A preferred technical solution of the solid dispersion in the present invention, and the compositions are as follows:

| Components | Content (unit) |
|---|---|
| Allisartan isoproxil | 1 |
| Povidone K29/32 | 0.2 |
| Crosslinked povidone | 0.4 |

A preferred technical solution of the solid dispersion in the present invention, and the compositions are as follows:

| Components | Content (unit) |
|---|---|
| Allisartan isoproxil | 1 |
| Povidone K29/32 | 0.3 |
| Crosslinked povidone | 0.05 |
| Microcrystalline cellulose | 0.30 |

A preferred technical solution of the solid dispersion in the present invention, and the compositions are as follows:

| Components | Content (unit) |
|---|---|
| Allisartan isoproxil | 1 |
| Hydroxypropyl methylcellulose phthalate (HP-55) | 0.4 |
| Crosslinked povidone | 0.35 |

It is another object of the present invention to provide a pharmaceutical composition of allisartan isoproxil. The mentioned pharmaceutical composition is composed of allisartan isoproxil solid dispersion of the present invention and pharmaceutically acceptable excipients. The pharmaceutically acceptable excipients can comprise one or mixture of more than one of disintegrant, binder, filler, lubricant, etc.

The disintegrant is selected from one or mixture of more than one of cross-linked sodium carboxymethyl cellulose, cross-linked povidone, sodium carboxymethyl starch, low-substituted hydroxypropyl cellulose, starch, pregelatinized starch, etc., the amount of disintegrant should be the same as known in the field which can achieve the effect of disintegrating. Preferably, the mass ratio of the solid dispersion to the disintegrant in mentioned pharmaceutical composition is 1: 0.02 to 0.20.

The binder can be added depending on the needs of pharmaceutical preparations; specifically, the binder is selected from one or mixture of more than one of hydroxypropyl methylcellulose, hydroxypropyl cellulose, sodium carboxymethyl cellulose, povidone, starch paste, gelatin, etc.. When adding the binder, the amount should be the same as known in the field which can achieve the effect of binding, preferably, the mass ratio of solid dispersion to the binder in a pharmaceutical composition is 1: 0.01 to 0.05.

The filler can be added depending on the needs of pharmaceutical preparations, specifically, the filler is selected from one or mixture of more than one of lactose, mannitol, dextrin, microcrystalline cellulose, starch, pregelatinized starch, calcium sulfate, calcium phosphate, calcium hydro phosphate, etc.. The amount of filler should be the same as known in the field which can achieve filling effect; preferably, the mass ratio of solid dispersion to the filler in a pharmaceutical composition is 1: 0.02 to 0.20. Mentioned lubricant is selected from one or mixture of more than one of magnesium stearate, colloidal silicon dioxide, talc, stearic acid, etc.. The amount of lubricant should be the same as known in the field which can achieve lubricating effect.

The pharmaceutical compositions of allisartan isoproxil can be tablets, capsules, granules, pills and other conventional oral preparations, preferably tablets and capsules. The pharmaceutical compositions should be prepared using common formulation means in the field, specifically, for tablets, dry granulation, wet granulation and direct compression method can be used; for capsules, dry granulation, wet granulation or direct-powder-fill method can be used.

For one specific example of allisartan isoproxil pharmaceutical composition in the present invention, its formulation and preparation methods are as follows:

| Components | Content (unit) |
|---|---|
| Solid dispersion | 1 |
| Microcrystalline cellulose | 0.10 |
| Lactose | 0.03 |
| Crosslinked povidone | 0.03 |
| Magnesium stearate | 0.01 |
| Opadry | 0.02 |

Mix the resulting solid dispersion and excipients, directly compress into tablets. If necessary, it can be further coated with premixed coating agent to obtain coated tablets. The allisartan isoproxil pharmaceutical composition in present invention can be used for the treatment of hypertension and its complications, preferably, mentioned allisartan isoproxil pharmaceutical composition can be used for treatment of mild to moderate primary hypertension. The complications of hypertension refer to the symptoms caused by hypertension, including cardiac complications, such as left ventricular hypertrophy, angina, myocardial infarction, heart failure; Sroke, such as hemorrhagic stroke, ischemic stroke, hypertensive encephalopathy; hypertensive renal damage, such as the slow progress of the small arteries of the kidney sclerosis, malignant small renal arterial sclerosis, chronic renal failure; ophthalmic diseases, such as retinal arteriosclerosis, retinal change.

The present invention has the following outstanding advantages and beneficial effects compared with available technologies:
1. Provides an allisartan isoproxil solid dispersion with high drug loading, characterized with obvious advantages in drug loading, dissolution, stability, etc.;
2. Provides a pharmaceutical composition of allisartan isoproxil containing allisartan isoproxil solid dispersion mentioned in the present invention, characterized with good dissolution performance, high stability and good compliance. It finally optimizes the clinical dose and the antihypertensive effect.

### Detailed description of the examples

As below the present invention will be described in further detail in conjunction with examples, but it is not limited to this.

### Example 1

**Formulation:**

| Type | Components | Content (mg/tab) |
|---|---|---|
| Solid dispersion | Allisartan isoproxil | 240 |
| | Povidone K29/32 | 84 |
| | Crosslinked povidone (I) | 84 |
| Extragranular material | Microcrystalline cellulose | 36 |
| | Crosslinked povidone (II) | 36 |
| | Magnesium stearate | 4.6 |
| Coating material | Opadry | 9.6 |
| Theoretical tablet weight | | 494.2 |

### Preparation:

### 1. Preparation of solid dispersion

Dissolved the drug and povidone K29/32 in a suitable amount of methylene chloride-ethanol mixed solution firstly, and then added crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Example 2

**Formulation:**

| Type | Components | Content (mg/tab) |
|---|---|---|
| Solid dispersion | Allisartan isoproxil | 240 |
| | Povidone K29/32 | 48 |
| | Crosslinked povidone (I) | 96 |
| Extragranular material | Microcrystalline cellulose | 37.2 |
| | Lactose | 11.2 |
| | Crosslinked povidone (II) | 11.2 |
| | Magnesium stearate | 3.7 |
| Coating material | Opadry | 8.9 |
| Theoretical tablet weight | | 456.2 |

### Preparation:

### 1. Preparation of solid dispersion

Dissolved the drug and povidone K29/32 in a suitable amount of methylene chloride-ethanol mixed solution firstly, and then added crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Example 3

**Formulation:**

| Type | Components | Content (mg/tab) |
|---|---|---|
| Solid dispersion | Allisartan isoproxil | 240 |
| | Povidone K29/32 | 72 |
| | Microcrystalline cellulose | 72 |
| | Crosslinked povidone(I) | 12 |
| Extragranular material | Crosslinked povidone (II) | 37.2 |
| | Magnesium stearate | 3.7 |
| Coating material | Opadry | 8.7 |
| Theoretical tablet weight | | 445.6 |

### Preparation:

### 1. Preparation of solid dispersion

Dissolved the drug and povidone K29/32 in a suitable amount of methylene chloride-ethanol mixed solution firstly, and then added microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Example 4

**Formulation:**

| Type | Components | Content (mg/tab) |
|---|---|---|
| Solid dispersion | Allisartan isoproxil | 240 |
| | PEG6000 | 60 |
| | Sodium carboxymethyl starch (I) | 24 |
| | Microcrystalline cellulose | 96 |
| Extragranular material | Sodium carboxymethyl starch (II) | 48 |
| | Magnesium stearate | 4.7 |
| Coating material | Opadry | 9.5 |
| Theoretical tablet weight | | 482.2 |

### Preparation:

### 1. Preparation of solid dispersion

Dissolved the drug and PEG6000 in a suitable amount of methylene chloride - ethanol mixed solution firstly, and then added microcrystalline cellulose and sodium carboxymethyl starch (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Example 5

**Formulation:**

| Type | Components | Content (mg/tab) |
|---|---|---|
| Solid dispersion | Allisartan isoproxil | 240 |
| | Copovidone S630 | 84 |
| | Microcrystalline cellulose | 108 |
| Extragranular material | Crosslinked povidone | 55 |
| | Magnesium stearate | 4.9 |
| Coating material | Opadry | 9.8 |
| Theoretical tablet weight | | 501.7 |

### Preparation:

### 1. Preparation of solid dispersion

Dissolved the drug and copovidone S630 in a suitable amount of methylene chloride - ethanol mixed solution firstly, and then added microcrystalline cellulose into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Example 6

**Formulation:**

| Type | Components | Content (mg/tab) |
|---|---|---|
| Solid dispersion | Allisartan isoproxil | 240 |
| | Hydroxypropyl cellulose SL | 90 |
| | Microcrystalline cellulose | 89 |
| Extragranular material | Low-substituted hydroxypropyl cellulose | 52 |
| | Magnesium stearate | 4.7 |
| Coating material | Opadry | 9.5 |
| Theoretical tablet weight | | 485.2 |

### Preparation:

### 1. Preparation of solid dispersion

Dissolved the drug and hydroxypropyl cellulose SL in a suitable amount of methylene chloride - ethanol mixed solution firstly, and then added microcrystalline cellulose into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Example 7

**Formulation:**

| Type | Components | Content (mg/tab) |
|---|---|---|
| Solid dispersion | Allisartan isoproxil | 240 |
| | HPMCP HP-55 | 96 |
| | Crosslinked povidone (I) | 84 |
| Extragranular material | Microcrystalline cellulose | 25 |
| | Crosslinked povidone (II) | 25 |
| | Magnesium stearate | 4.7 |
| Coating material | Opadry | 9.5 |
| Theoretical tablet weight | | 484.2 |

### Preparation:

### 1. Preparation of solid dispersion

Dissolved the drug and HPMCP HP-55 in a suitable amount of methylene chloride-ethanol mixed solution firstly, and then added crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Comparative Example 1

**Formulation:**

| Type | Components | Content (mg/tab) |
|---|---|---|
| Solid dispersion | Allisartan isoproxil | 240 |
| | Povidone K29/32 | 128 |
| | Crosslinked povidone (I) | 320 |
| Extragranular material | Crosslinked povidone (II) | 40 |
| | Lactose | 160 |
| | Stearic acid | 6.4 |
| Coating material | Opadry | 17.9 |
| Theoretical tablet weight | | 912.3 |

### Preparation:

### 1. Preparation of solid dispersion

Dissolved the drug and povidone K29/32 in a suitable amount of methylene chloride-ethanol mixed solution firstly, and then added crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the solid dispersion, proving the desired effect was achieved.

### 2. Preparation of the pharmaceutical composition

Mixed the solid dispersion with the remaining materials, compressed into tablets, performed film coating and finally obtained an allisartan isoproxil pharmaceutical composition.

### Comparative Example 2

### Formulation:

| Type | Components | Content (mg/tab) |
|---|---|---|
| Solid dispersion | Allisartan isoproxil | 240 |
| | Povidone K29/32 | 36 |
| | Microcrystalline cellulose | 72 |
| | Crosslinked povidone (I) | 12 |
| Extragranular material | Crosslinked povidone (II) | 33.8 |
| | Magnesium stearate | 3.9 |
| Coating material | Opadry | 8.0 |
| Theoretical tablet weight | | 405.7 |

### Preparation:

### 1. Preparation of solid dispersion

Dissolved the drug and povidone K29/32 in a suitable amount of methylene chloride-ethanol mixed solution firstly, and then added microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the form of crystal in the solid dispersion.

### 2. Preparation of the pharmaceutical composition

Mixed the solid dispersion with the remaining materials, compressed into tablets, performed film coating and finally obtained an allisartan isoproxil pharmaceutical composition.

### Comparative Example 3

**Formulation:**

| Type | Components | Content (mg/tab) |
|---|---|---|
| Solid dispersion | Allisartan isoproxil | 240 |
| | POLYOX WSR N-10 NF (polyoxyethylene) | 72 |
| | Microcrystalline cellulose | 72 |
| | Crosslinked povidone (I) | 12 |
| Extragranular material | Crosslinked povidone (II) | 4.0 |
| | Magnesium stearate | 4.0 |
| Coating material | Opadry | 8.1 |
| Theoretical tablet weight | | 412.1 |

### Preparation:

### 1. Preparation of solid dispersion

Dissolved the drug and POLYOX WSR N-10 NF in a suitable amount of methylene chloride-ethanol mixed solution firstly, and then added microcrystalline cellulose and crosslinked povidone (I) into the fluidized bed, sprayed the solution prepared into a fluidized bed granulator from top using a spray gun, and dried to obtain allisartan isoproxil solid dispersion; further XRD testing showed that allisartan isoproxil was highly dispersed in the form of crystal in the solid dispersion.

### 2. Preparation of the pharmaceutical composition

Mixed the solid dispersion with the remaining materials, compressed into tablets, performed film coating and finally obtained an allisartan isoproxil pharmaceutical composition.

### Example 8

Tested according to the second method in Appendix XC of the Chinese Pharmacopoeia in the dissolution test. Tested the dissolution of allisartan isoproxil pharmaceutical compositions obtained in Examples 1 to 7 and Comparative Examples 1 to 3 in pH6.8 phosphate buffer (37°C, dissolution medium 900mL, speed 50rpm) respectively. Sample at 15min, 30min and 45min. Test by UV spectrophotometer.

| Example | 15min | 30min | 45min |
|---|---|---|---|
| Example 1 | 80% | 88% | 95% |
| Example 2 | 71% | 85% | 94% |
| Example 3 | 86% | 90% | 96% |
| Example 4 | 80% | 85% | 92% |
| Example 5 | 85% | 90% | 97% |
| Example 6 | 71% | 85% | 95% |
| Example 7 | 84% | 91% | 95% |
| Comparative Example 1 | 85% | 93% | 96% |
| Comparative Example 2 | 65% | 73% | 80% |
| Comparative Example 3 | 70% | 76% | 84% |

As can be seen from the data above, allisartan isoproxil pharmaceutical compositions obtained in Examples 1 to 7 could reach dissolution of more than 90% in 45 minutes. Although the dissolution of Comparative Example 1 was good, the unit weight of the preparation was too large and patient compliance was poor due to excessive solubilizing carrier used.

The dissolution of Comparative Examples 2 and 3 did not meet the requirement of clinical medication. Specifically, too little of solubilizing carrier was added in Comparative Example 2, so active ingredient exists in the solid dispersion in the form of crystal, and preparation dissolution was affected; for Comparative Example 3, solubilizing carrier mentioned in the present invention was not used, so active ingredient exists in the solid dispersion in the form of crystal, and preparation dissolution was affected.

In the further study on the storage stability of allisartan isoproxil pharmaceutical compositions obtained in Examples 1∼7 for 6 months and 12 months, no significant change for active ingredient content were found, and the impurity contents were not increased significantly, so we could conclude that the pharmaceutical compositions obtained were with high stability.

The above examples are preferable examples of the present invention, but the detailed description is not restricted by the examples; other change, modification, substitution, combination, simplification and any other departure from the spirit and principle of the present invention are considered as equivalent replacement, and should be included within the protection of the invention.

## Claims

1. An allisartan isoproxil solid dispersion composed of allisartan isoproxil and pharmaceutically acceptable carrier materials, wherein the carrier materials comprise solubilizing carrier, **characterized in that** the mass ratio of allisartan isoproxil to the solubilizing carrier in mentioned allisartan isoproxil solid dispersion is 1: 0.2 ∼ 0.45.

2. An allisartan isoproxil solid dispersion according to claim 1, wherein the solubilizing carrier is selected from one or mixture of two or more in any ratio of vinylpyrrolidone homopolymers or copolymers, polyvinyl alcohol, polyethylene glycol, cellulose ether, acrylic polymer, hydroxypropyl methyl cellulose phthalate, cellulose acetate phthalate, hydroxypropyl methylcellulose acetate succinate.

3. An allisartan isoproxil solid dispersion according to any one of claims 1 to 2, wherein the solubilizing carrier in mentioned allisartan isoproxil solid dispersion is one or mixture of two or more in any ratio of povidone, copovidone, polyethylene glycol, hydroxypropyl cellulose, hydroxypropyl methyl cellulose, hydroxypropyl methyl cellulose phthalate.

4. An allisartan isoproxil solid dispersion according to any one of claim 1 to 3, wherein the mass ratio of allisartan isoproxil to the solubilizing carrier in mentioned allisartan isoproxil solid dispersion is 1: 0.30 ∼ 0.40.

5. An allisartan isoproxil solid dispersion according to any one of claim 1 to 4, wherein the mentioned allisartan isoproxil solid dispersion further contains excipients, and the mass ratio of allisartan isoproxil to the excipients is 1:0.1 ∼ 1.0.

6. An allisartan isoproxil solid dispersion according to any one of claim 1 to 4, wherein the mentioned allisartan isoproxil solid dispersion further contains excipients, and the mass ratio of allisartan isoproxil to the excipients is 1:0.3 ∼ 0.8.

7. An allisartan isoproxil solid dispersion according to any one of claim 5 to 6, wherein the mentioned excipients are selected from one or mixture of two or more in any ratio of cross-linked povidone, cross-linked sodium carboxymethyl cellulose, low-substituted hydroxypropyl cellulose, sodium carboxymethyl starch, microcrystalline cellulose, starch, pre-gelatinized starch, lactose, dextrin, mannitol, calcium sulfate, calcium phosphate, calcium hydrogen phosphate.

8. An allisartan isoproxil pharmaceutical composition, wherein the pharmaceutical composition comprises allisartan isoproxil solid dispersion according to any one of claim 1 to 7 and pharmaceutically acceptable excipients.

9. An allisartan isoproxil pharmaceutical composition according to claim 8, wherein the mentioned pharmaceutically acceptable excipients comprise one or mixture of two or more of disintegrant, binder, filler, lubricant; the mentioned disintegrant is one or mixture of two or more of crosslinked sodium carboxymethyl cellulose, cross-linked povidone, sodium carboxymethyl starch, low-substituted hydroxypropyl cellulose, starch, pre-gelatinized starch or a mixture of two or more; the mentioned binder is one or mixture of two or more of hydroxypropyl methylcellulose, hydroxypropyl cellulose, sodium carboxymethyl cellulose, povidone, starch slurry, gelatin; the mentioned filler is one or mixture of two or more of lactose, mannitol, dextrin, microcrystalline cellulose, starch, pregelatinized starch, calcium sulfate, calcium phosphate, calcium hydrogen phosphate; the mentioned lubricant is one or mixture of two or more in any ratio of magnesium stearate, colloidal silicon dioxide, talc, stearic acid.

10. An allisartan isoproxil pharmaceutical composition according to claim 9, wherein the mass ratio of solid dispersion to the disintegrant in the pharmaceutical composition is 1: 0.02 ∼ 0.20; the mass ratio of solid dispersion to the binder is 1: 0.01 to 0.05; the mass ratio of solid dispersion to the filler is 1: 0.02 ∼ 0.20.

11. An allisartan isoproxil pharmaceutical composition according to any one of claim 8 to 10, wherein the mentioned allisartan isoproxil pharmaceutical composition can be tablets, capsules, granules or pills.

12. An allisartan isoproxil pharmaceutical composition according to any one of claim 8 to 11, wherein the mentioned allisartan isoproxil pharmaceutical composition can be used for the treatment of hypertension and its complications.
